# EUROPEAN PATENT SPECIFICATION

(11) **EP 1 930 002 B1**
(45) Date of publication and mention of the grant of the patent: **06.01.2016**
(21) Application number: 06761527.8
(22) Date of filing: 21.07.2006
(51) Int. Cl.: A61K 9/46, A61K 9/00, A61K 9/107, A61K 31/215, A61K 31/216

(54) **USE OF HYDROXYBENZOIC ACID ESTER COMPOUNDS FOR THE MANUFACTURE OF A MEDICAMENT FOR THE PREVENTION AND TREATMENT OF HPV INFECTION**
VERWENDUNG VON HYDROXYBENZOESÄURE-ESTER VERBINDUNGEN ZUR HERSTELLUNG EINES MEDIKAMENTS ZUR PRÄVENTION UND BEHANDLUNG VON HPV-INFEKTIONEN
UTILISATION DES ESTERS D'ACIDE HYDROXYBENZOÏQUE POUR LA FABRICATION D'UN MÉDICAMENT POUR LA PRÉVENTION ET LE TRAITEMENT DES INFECTIONS PAR HPV

(30) Priority: 02.08.2005 CN 200510012287
(43) Date of publication of application: 11.06.2008
(73) Proprietor: SHENGHUA (GUANGZHOU) PHARMACEUTICAL SCIENCE &, No.102 Xianlie Zhong Road, Guangzhou Guangdong 510070 (CN)
(72) Inventor: QIN, Weihua, F/22 NorthTower, Guangzhou, Guangdong 510070 (CN)
(74) Representative: Vandeberg, Marie-Paule L.G.
(86) International application number: PCT/CN2006/001792
(87) International publication number: WO 2007/014515

(56) References cited:
- WO-A1-96/24367
- WO-A1-02/089818
- CN-A- 1 167 567
- CN-C- 1 068 782
- DE-A1- 19 514 694
- US-A- 3 929 985
- US-B1- 6 440 428
- DATABASE WPI Week 199719 Thomson Scientific, London, GB; AN 1997-209221 XP002572980 & JP 09 059151 A (KAO CORP) 4 March 1997 (1997-03-04)
- DATABASE WPI Week 198729 Thomson Scientific, London, GB; AN 1987-203555 XP002572971 & JP 62 132824 A (EISAI CO LTD) 16 June 1987 (1987-06-16)
- SONI M G ET AL: "Safety assessment of esters of p-hydroxybenzoic acid (parabens)" FOOD AND CHEMICAL TOXICOLOGY, PERGAMON, GB, vol. 43, no. 7, 1 July 2005 (2005-07-01), pages 985-1015, XP004839899 ISSN: 0278-6915
- KANE C.-J. ET AL.: 'Methyl gallate, methyl-3,4,5-trihydroxybenzoate, is a potent and highly specific inhibitor of herpes simplex virus in vitro. II. Antiviral activity of methyl gallate and its derivatives' BIOSCI.-REP. vol. 8, no. 1, February 1988, pages 95 - 102, XP003008356
- ZHENG S. ET AL.: 'The relationship of cervial cancer with pathogen infections, cytokine and Se' CHINESE J. EXP. CLIN. VIROL. vol. 16, no. 2, June 2002, pages 179 - 183, XP001249213

## Description

### Technical Field

This invention is related to the use of hydroxybenzoic acid esters for the manufacture of a medicament for the prevention and treatment of virus infection. Particularly, the invention is related to the use of hydroxybenzoic acid esters for the manufacture of a medicament for the prevention and treatment of human or animal HPV infection, condyloma acuminata induced by HPV, cervical erosion in HPV positive patients, chronic cervicitis in HPV positive patients, or to aid in the treatment of cervical cancer.

### Technical Background

Among hydroxybenzoic acid esters and analogues thereof, p-hydroxybenzoic acid esters were often used as food and drug preservatives. Gallic acid ester ("3, 4, 5,-trihydroxybenzoic acid ester") can inhibit the synthesis of thromboxane A2 (TXA2). It has a stronger and faster effect against platelets aggregation than aspirin (ASP) and has been used in solution for injection. Gallic acid has been proven to inhibit hepatitis B virus replication (see Journals of Guangzhou Liberation Army High Specialized School, 1998, 26 (2):5-7). So far there has been no report of using hydroxybenzoic acid esters and analogues thereof including 3, 4, 5,-trihydroxybenzoic acid esters to treat virus infection in human, for example, hepatitis B or skin mucosa virus infection.

Chinese Patent No. CN 1411339A and U. S. Patent No. 6,294,186 disclosed an antimicrobial composition. The composition includes a safe and effective amount of benzoic acid analogues, which have the following structure (II):

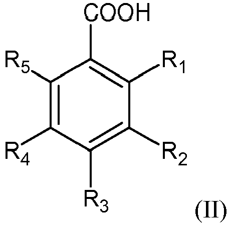

Wherein, R₁, R₂, R₄ and R₅ were independently H, OH, F, I, Br, Cl, SH, NH₂, CN, alkyl, alkoxy, NR₂, OR, NO₂, COR, CONR₂, CO₂R or SO₃R, wherein R was independently H, alkyl or alkoxy; R₃ was independently H, OH, F, I, Br, Cl, SH, CN, alkyl, alkoxy, OR, NO₂, COR, CONR₂, CO₂R or SO₃R, wherein R was independently H, alkyl or alkoxy. The aforesaid patent references also pointed out the most preferred embodiment as salicylic acid ("2-hydroxybenzoic acid"), benzoic acid or their combination; they also limited the pH range in which these compounds could be used to 1-7.

International patent No. WO 96/24367 disclosed a pharmaceutical composition comprising propyl gallate as antiviral agent.for the treatment of herpes simplex virus infection (HSV-1).

Japanese patent JP 09 059151 disclosed a composition comprising gallate such as methyl gallate, in the treatment of HIV.

German patent DE 195 14 694 disclosed a composition comprising 4-hodroxybenzoic acid esters for the treatment and prevention of viral infections such as hepatitis B and herpes simplex.

Japanese patent JP 62 132824 disclosed a composition comprising ethyl gallate for the treatment of hepatitis

Kane C-J et A1 disclosed the antiviral activity of methyl gallate against herpes viruses.

Although viruses are the smallest known pathogenic microorganisms, they spread most widely. Today, almost three quarters of contagious diseases in the world are caused by viruses. In clinical diagnosis, many diseases are related to virus infection.

Condyloma acuminata, also called genital warts or venereal warts, is a benign skin mucosa growth induced by human papillomavirus (HPV). It is one of the most frequently seen sexually transmitted diseases and is closely related to inflammation and cancer of the genital organs. Currently, podophyllotoxin is one of the drugs for treating genital warts. Its advantage is short treatment time and disadvantage is skin irritation and severe side effects, including pain, edema, erosion and so on. Recombinant interferon α-2β gel is recognized as an effective external drug to treat genital warts.

Recent researches indicated that 29.3% of the patients of cervical erosion were tested HPV positive while the positive rate among the normal population is only 11.1% (Fuqiang Chen, Tumor Prevention Magazine, 2001, 8 (4) 342-344; Chenkang Xu, Journal of Zhongshan Medical University, 1998, 19 (3): 223-226). It was reported that the high-risk HPV 16 and HPV 18 were expressed in about 69% of chronic cervicitis patients; and that these subtypes were detected at increasing rates as the degree of the cervical erosion increased (Ahn ws et al, J Cell Biochem Suppl, 1997; 28-29: 133-139). The difference of the detection rates of HPV 16 and HPV18 between granular or papillary erosion and normal cervix was significant. The positive rate of HPV 16 and HPV18 in cervix cancer could be as high as 83.33% when detected by PCR. The DNA of high risk HPV could integrate into the chromosomes of the host cells and then produce E6 and E7 tumor proteins. E6 and E7 could respectively inhibit anti-cancer genes p53 and Rb. As a result, cells would lose the control of p53 and Rb. This would cause cells in the steady state to grow actively and might become cancerous. If HPV16 remains in the cells, it could turn the cervix pathological changes into cancer. Different from the pudendum infection, cervix HPV infection mainly involves HPV 16/18 and usually will not cause warts. It will exist as latent infection for a long time and induce atypical hyperplasia at first and become cancerous when the other factors add on.

So, people are always researching and developing new drugs for preventing and treating HPV infection, condyloma acuminata induced by HPV, cervical erosion in HPV positive patients, chronic cervicitis in HPV positive patients or to aid in the treatment of cervical cancer.

### Description of the invention

The main purpose of this invention is to provide the use of hydroxybenzoic acid esters for the manufacture of a medicament for the prevention and treatment of HPV infection and related diseases in human and animals.

The present invention provides hydroxybenzoic acid esters illustrated by the following general Formula (I):

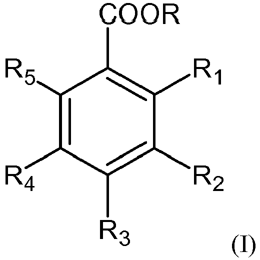

Wherein, R is C₁₋₁₁ alkyl; R₁, R₂, R₃, R₄, R₅ are independently OH or H, and at least two of which are OH.

Preferably, R is C₁₋₈ alkyl, i. e. CH₃, C₂H₅, C₃H₇, C₄H₉, C₅H₁₁, C₆H₁₃, C₇H₁₅, C₈H₁₇; the most preferred number of carbon atoms is 1-3, i. e. CH₃, C₂H₅ and C₃H₇.

R₁, R₂, R₃, R₄, R₅ together contain two or more OH groups, and further preferably contain three or more OH groups.

In certain preferred embodiments, the hydroxybenzoic acid esters of Formula (I) include the following compounds: 2, 4-dihydroxybenzoic acid ester; 3, 4- dihydroxybenzoic acid ester; 2, 3, 4- trihydroxybenzoic acid ester; 3, 4, 5- trihydroxybenzoic acid ester or 3, 4, 6- trihydroxybenzoic acid ester. In a preferred embodiment, the compounds of the invention include 2, 3, 4- trihydroxybenzoic acid ester, 3, 4, 5- trihydroxybenzoic acid ester and 3, 4, 6- trihydroxybenzoic acid ester. In a most preferred embodiment, the compounds of the invention include 3, 4, 5-trihydroxybenzoic acid ester.

The compounds of the present invention and/or compositions containing such compounds as the active ingredients can be used to manufacture medicaments for the treatment of virus-related diseases in human and animals, in particular for the prevention and treatment of HPV infection and related diseases, i.e. condyloma acuminata induced by HPV, cervical erosion in HPV positive patients, chronic cervicitis in HPV positive patients, or to aid in the treatment of cervical cancer.

As therapeutic drugs, the compounds of the present invention and/or compositions containing an effective amount of such compounds as active ingredients are the main components of the products for preventing and treating virus infection in human bodies and skins. It is not just used as the adjuvant ingredients such as preservatives, antioxidants and so on. If the antivirus effect of a product declines as the concentration of the compounds and/or compositions containing such compounds as an active ingredients declines, it shall have acted as the effective ingredient for the prevention and treatment of skin mucosa virus related diseases. The aforesaid effective amount means the dose at which such compounds or compositions can effectively eliminate or prevent virus infection.

Most of the compounds of the present invention are known compounds. Some of them have been used to make food additives, some are pharmaceutical adjuvants and some are pharmaceutical intermediates which can be purchased on the market, extracted from natural plants or made by known methods.

The compounds of the present invention and/or compositions containing such compounds, together with various adjuvants for injection or oral formulations, can be made into powder, solution, tablets, capsules, granules, liquid, or other pharmaceutically acceptable preparations.

The compounds of the present invention and/or compositions containing such compounds, together with various adjuvants for skin mucosa formulations, can be made into various preparations, such as ointments, gels, wash liquids, tablets, suppositories, diaphragms, sprays or other pharmaceutically acceptable preparations.

The compounds of the present invention and/or compositions containing such compounds can be used to make antivirus personal care products. Personal care products include, but are not limited to, sterilization solutions, hand soaps, hand sanitizers, body cleansers, body cleansing gels, body washing soaps, personal care wipes, facial tissues, nasal sprays.

A single compound or a combination of more than one compound of the present invention can be used for manufacturing the medicaments of this invention. The compound(s) can be combined with different pharmaceutically acceptable carriers to facilitate the formation of a pharmaceutically acceptable formulation. These carriers include but are not limited to starch, dextrine, Tween-80, Carbomer, cellulose, carboxy methyl cellulose sodium alginate, glycerin, propanediol, polyethylene glycol, laurocapram, isopropanol, trolamine and Span 60 (sorbitan monostearate).

In addition, while a single compound or combination of the compounds are active ingredients, the active ingredients can be added into sterilization solutions, hand soaps, hand sanitizers, body cleansers, body cleansing gels, body washing soaps, personal care wipes, facial tissues or nasal sprays to kill viruses on skin.

The formulations and personal care products of this invention can be made with conventional methods known in this field.

The compounds and/or compositions containing such compounds can effectively kill or prevent HPV 6/11, 16/18 viruses, can be used to manufacture medicaments for treatment of HPV virus infection, to treat condyloma acuminata, vaginitis, cervicitis, cervical erosion and to aid in the treatment of cervical cancer. This invention uses real time quantitative fluorescent PCR to detect the amount of HPV in the sample to screen the compounds and their treatment effects. This method has been reported in several publications (Youling, Cai, Prevention of Chinese venereal disease and cancer, 2002, 8:2, 108; Yuansheng, Wu, Ruiqiang, Qu, Practical Chinese and western medicine clinics, 2003, 3: 2, 1).

Some of the compounds of the present invention are food additives which are nontoxic in its dose range. Clinical testing of patients infected by HPV or HSV has shown that hydroxybenzoic acid esters and analogous thereof have better curative effect on virus infection than hydroxybenzoic acid and analogues thereof.

Hydroxybenzoic acid esters are more stable than hydroxybenzoic acids. Experiments showed that propyl gallate was more stable than gallic acid, especially when it is in weak alkaline condition of the human body (pH 7.4) or intestinal alkaline condition (pH 8.6). Hydroxybenzoic acid solution changed from colorless to yellow in a period of time shorter than hydroxybenzoic acid ester under the condition of pH 6.8~8.6, as shown by spectrophotometry.

Meanwhile, the antiviral activity of a hydroxybenzoic acid ester is higher than that of its corresponding acid. For example, the antiviral activity of propyl gallate is about one time higher than that of gallic acid. The data shown in the examples of this application demonstrated that hydroxybenzoic acid esters did not take effect as the prodrugs of hydroxybenzoic acids. Therefore, the compounds of the present invention can be made into antiviral drugs or personal care products with the advantages of controlled quality and remarkable curative effect.

### Examples

The following examples are provided to further illustrate the present invention and are not to be interpreted as limiting the scope of the invention.

The materials (>99% of content) used in the present invention and their suppliers are as follows:

| Materials | Suppliers |
|---|---|
| p-hydroxybenzoic acid | Sinopharm Chemical Reagent Co., Ltd. |
| Ethyl p-hydroxybenzoate | GuangZhou Gases Factory Co., Ltd. |
| 2,3-dihydroxybenzoic acid | Taizhou Zhongda Chemical Co., Ltd. |
| Methyl 2,3-dihydroxybenzoate | Taizhou Zhongda Chemical Co., Ltd. |
| 2,4-dihydroxybenzoic acid | Taizhou Zhongda Chemical Co., Ltd. |
| Methyl 2,4-dihydroxybenzoate | Taizhou Zhongda Chemical Co., Ltd. |
| 3,4-dihydroxybenzoic acid | Taizhou Zhongda Chemical Co., Ltd. |
| Methyl 3,4-dihydroxybenzoate | Taizhou Zhongda Chemical Co., Ltd. |
| 2,3,4-trihydroxybenzoic acid | Nanjing Longyuan Natural Polyphenol Synthesis Factory |
| Gallic acid | Zhejiang Ouhai Chemical Reagent Co. Ltd. |
| Propyl gallate | Lianyungang Hongqi Chemical Plant |
| Ethyl gallate | Nanjing Longyuan Natural Polyphenol Synthesis Factory |
| Methyl gallate | Nanjing Longyuan Natural Polyphenol Synthesis Factory |
| Octyl gallate | Nanjing Longyuan Natural Polyphenol Synthesis Factory |
| Dodecyl gallate | Nanjing Longyuan Natural Polyphenol Synthesis Factory |
| Recombinant human interferon α-2β gel | SIU-FUNG USTC Pharmaceutical Co., Ltd. |
| Polyinosinic acid | Guangdong Kaiping Biochemical Pharmaceutical Co., Ltd. |

### Example 1. Preparation of anti-HPV micro emulsion

20 ml 1,2-propylene glycol, 15 ml Tween-80 and 5 ml azone were mixed together, proper amount of sterile distilled water was added to obtain 100 ml of solution for external use. 10 g each of benzoic acid, salicylic acid, a combination of benzoic acid and salicylic acid, p-hydroxybenzoic acid, 2, 3-dihydroxybenzoic acid, 2, 4-dihydroxybenzoic acid, 3, 4-dihydroxybenzoic acid, ethyl p-hydroxybenzoate, methyl 3, 4-dihydroxybenzoate, gallic acid, dodecyl gallate, octyl gallate, propyl gallate, ethyl gallate, methyl gallate, methyl 2, 4-dihydroxybenzoate were added into 50 ml of the solution for external use, respectively. The pH value of the solution was adjusted to 5.5. Then proper amount of the solution for external use was added to obtain 100 ml micro emulsion containing 10% of the compounds of the present invention or the control group.

### Example 2. Preparation of anti-HBV injection solution containing propyl gallate

10 g propyl gallate, 8.5 g sodium chloride, 10 ml 1, 2-propylene glycol and 10 ml Tween-80 were mixed and dissolved in sterile distilled water to a final volume of 100 ml. The pH value of the solution was adjusted to 7.4. Then the solution was filtrated, encapsulated and sterilized at 100 °C for 30 minutes to obtain the solution for injection of propyl gallate.

### Example 3. Preparation of effervescent tablets containing ethyl gallate

0.25 g ethyl gallate, 0.01 g polyinosinic acid and 0.45 g tartaric acid were sifted through a 80 mesh sieve separately, made into soft materials with 95% ethanol, then sifted through a 12 mesh sieve. The obtained wet particles were dried at 50 °C. 0.65 g sodium hydrogen carbonate, 0.02 g dextrin and sterile distilled water were mixed together to produce soft material, which was then sifted through a 12 mesh sieve and dried at 50 °C. The obtained materials were mixed with the dried particles produced initially. Finally, sterile distilled water was added into the mixture, heated, mixed with 0.01 g PEG6000 and made into effervescent tablets.

### Example 4. Drug screening through quantitative fluorescent PCR analysis

### Samples

The micro emulsions obtained in Example 1 were used as test samples, and saline water was used as the control.

HPV samples: condyloma acuminata samples were taken from clinical patients. The samples were washed with saline water to wash away the blood. Then they were cut into pieces under sterile condition, mixed with three times volume of saline water, homogenized and stored at -40°C for further use.

**Table 1 Test result for drug screening by quantitative fluorescent PCR analysis of HPV**

| Test compounds | HPV 6/11 Virus (copies/ml) | HPV 16/18 Virus (copies/ml) |
|---|---|---|
| Control | 1.45×10⁶ | 1.40×10⁵ |
| Benzoic acid | 3.60×10⁵ | 2.90×10⁴ |
| Salicylic acid | 5.50×10⁵ | 2.60×10⁴ |
| Benzoic acid and Salicylic acid | 2.90×10⁵ | 4.50×10⁴ |
| p-hydroxybenzoic acid | 7.00×10⁵ | 1.30×10³ |
| Ethyl p-hydroxybenzoate | 8.50×10⁴ | 0 |
| 3,4-dihydroxybenzoic acid | 1.90×10⁴ | 8.50×10⁴ |
| Methyl3, 4-dihydroxybenzoate | 8.50×10⁴ | 6.00×10² |
| Gallic acid | 0 | 0 |
| dodecyl gallate | 6.70×10⁴ | 4.90×10⁴ |
| Octyl gallate | 0 | 0 |
| Propyl gallate | 0 | 0 |
| Ethyl gallate | 0 | 0 |
| Methyl gallate | 0 | 0 |
| 2,3-dihydroxybenzoic acid | 2.30×10⁴ | 3.20×10³ |
| 2,4-dihydroxybenzoic acid | 1.60×10⁴ | 4.60×10³ |
| Methyl 2, 4-dihydroxybenzoate | 5.20×10⁴ | 2.50×10² |

### Experiment methods

50 µl micro emulsion of each test compound or 50 µl saline water was added into 50 µl homogenate of a HPV sample. The mixture was incubated in warm bath at 37°C for 24 hours. Then the amount of HPV 6/11 and HPV 16/18 DNA was detected using FQ-PCR diagnostic kit purchased from Da An Gene Diagnostics Center. 0. 2 ml DNA was extracted from each sample by conventional alkali cleavage method and put into a thin wall tube. Proper amount of primers, F-PROBE, DNTP, DNA Polymerase and buffer solutions were added to the tube. ABI PRISM^{™}7700 quantitative fluorescent PCR machine was used. The reaction was conducted under the following conditions: initial denature at 93 °C for 2 minutes; further denature and anneal at 93 °C for 45 seconds and 55 °C for 120 seconds, repeat the cycle for 40 times. Finally, the result was analyzed and calculated by computer and shown in Table 1.

### Experiment results

24 hours after adding the test compound, no HPV 6/11 or HPV 16/18 was detected by FQ-PCR when octyl gallate, propyl gallate, ethyl gallate, methyl gallate, gallic acid was tested. For the group of ethyl p-hydroxybenzoate, HPV 16/18 was not detected, either. But HPV 16/18 was detected in the test with p-hydroxybenzoic acid. A small amount of HPV 16/18 was detected in the tests with methyl 2, 4-dihydroxybenzoate and methyl 3, 4-dihydroxybenzoate. On the contrary, high amounts of HPV 6/11 and HPV 16/18 were found in the control group and the tests with benzoic acid, salicylic acid and mixture of benzoic acid and salicylic acid, dodecyl gallate and 3, 4-dihydroxybenzoic acid. The results showed that the compounds of the present invention could eliminate HPV effectively and rapidly *in vitro.* The number of carbon atoms of the alcohol alkyl of hydroxybenzoic acid ester should be less than twelve.

### Example 5. Clinical use of hydroxybenzoic acid ester in treating condyloma acuminata

### Diagnosis standards

The following standards were taken from the Handbook for Venereal Disease Prevention and Treatment, the second edition, published by the Health and Epidemic Prevention Bureau of the Ministry of Health of the People's Republic of China.

Soft hyperplasia with nipple or cauliflower shaped or other shaped warts were found around the skin of the cunnus or anus. The result from 5% acetate acid white test was positive.

### Patients selection standards

Patients selected for the clinical observation must meet the above standards. Plus the diameter of their skin lesions should equal to or be less than 1 cm. Additionally, the patients did not have any treatment for the disease in the two weeks before the observation. Patients who had fungus, trichomoniasis, bacteria infection or other complications, chronic diseases, serious liver and kidney diseases or women in pregnancy or lactation were not selected. Treatments to women would not be conducted in their menstrual periods.

### Experiment methods

50 qualified patients were selected and randomly divided into five groups to test with p-hydroxybenzoic acid, gallic acid, ethyl p-hydroxybenzoate, propyl gallate and recombinant human interferon α-2β gel, respectively. The samples of p-hydroxybenzoic acid, gallic acid, ethyl p-hydroxybenzoate and propyl gallate were prepared as described in Example 1. The patients were treated with the test sample to the affected areas three times a day, in the morning, at noon and night. The samples were applied to cover the warts and the patients were required to leave the treated areas exposed and not to move for 20 minutes.

### Criteria for treatment effect:

Observation time: 8 days. Patients not cured after 8 days continued to use the test samples for another 12 weeks, and their prognosis was followed during the 12-week period.
Observed items: the location, shape, size and number of warts were carefully observed and noted before the treatment.
"Recovered": all skin lesions have disappeared;
"Obvious Effect": more than 70% of the skin lesions have disappeared;
"Improved": more than 30% of the skin lesions have disappeared;
"Not Effective": less than 30% of the skin lesions have disappeared or the original skin lesions didn't change or even enlarged.

The results showed that the recombinant human interferon α-2β gel which was regarded as the best drug in treating condyloma acuminata still took at least 4 weeks to eliminate the warts. Statistical analysis showed that the effect of ethyl p-hydroxybenzoate and propyl gallate was significantly better than that of p-hydroxybenzoic acid and gallic acid. When compared to recombinant human interferon α-2β gel, ethyl p-hydroxybenzoate and propyl gallate had more apparent effect. Additionally, the compounds of the present invention did not cause any obvious skin irritation. The only side effect was that some patients might occasionally experience a burning sense when the compounds were used.

| Table 2. Results of clinical use of compounds for treatment of condyloma acuminata | | | | | | | |
|---|---|---|---|---|---|---|---|
| | Recovered | | | Obvious Effect | | Improved | Not Effective |
| | 1-4 days | 5-8 days | | 1-4 days | 5-8 days | | |
| Gallic Acid | 2 | | | 3 | 1 | 4 | |
| Propyl Gallate | 7 | 1 | | 2 | | | |
| P-hydroxybenzoic Acid | | | | | 5 | 2 | 3 |
| Ethyl p-hydroxybenzoate | 6 | 2 | | 2 | | | |
| Recombinant Human Interferon α-2β Gel | The shortest period of treatment is 4 weeks | | | | | | |

Myristyl sodium sulfate, 1,2-propylene glycol, 4-chloro-3, 5-dimethylphenol, triclosan and fragrance material were mixed together according to the ratio listed in Table 4 below and water was added to the mixture to 5%. Then the mixture was heated to 80°C with stirring until the ingredients were fully dissolved. The resultant solution was cooled to room temperature. Ethyl p-hydroxybenzoate was added and dissolved in the solution with stirring. The pH value of the solution was adjusted to 5.0 with NaOH or HCl. Then sterile distilled water was added to obtain the product.

**Table 4. Preparation of liquid hand cleanser**

| Ingredients (wt.%) | 6 | 7 | 8 | 9 | 10 |
|---|---|---|---|---|---|
| Myristyl sodium sulfate | -- | 5.00 | 5.00 | -- | 5.00 |
| 1,2-propylene glycol | 20.00(V/V) | 20.00(V/V) | 20.00(V/V) | 20.00(V/V) | 20.00(V/V) |
| Ethyl p-hydroxybenzoate | 1.00 | 5.00 | 8.00 | 4.00 | -- |
| NaOH / HCl | To pH5.0 | To pH4.5 | To pH4.0 | To pH6.5 | To pH6.0 |
| 4-chloro-3, 5-dimethylphenol | 1.50 | -- | -- | -- | -- |
| Triclosan | -- | 0.25 | 1.00 | -- | 1.00 |
| Fragrance material | 1.0 | 1.0 | -- | 1.0 | 1.0 |
| Sterile distilled water | To 100% | To 100% | To 100% | To 100% | To 100% |

### Example 9. Stability test of propyl gallate and gallic acid

20 ml 1, 2-propylene glycol, 15 ml Tween-80 and 5 ml azone were mixed together and sterile distilled water was added to bring the volume to 100 ml. The pH value of the solution was adjusted to pH 6.8 with NaOH or HCl to obtain a micro emulsion. 10 g propyl gallate and 10 g gallic acid were dissolved in the micro emulsion, respectively, and mixed well. 10 ml of each micro emulsion was added to a capped transparent glass tube. Both solutions were colorless. The absorbance of the two solutions at 400-500 nm was measured with spectrophotometry and their absorbance was under 0.15. After the two solutions were kept at room temperature for 30 days, the gallic acid solution changed to light brown while the propyl gallate solution remained colorless. Measured by spectrophotometry again, the absorbance of the gallic acid solution was 1.2 and that of the propyl gallate solution was 0.4. It showed that propyl gallate was more stable in solution than gallic acid.

### Industrial Utility

The hydroxybenzoic acid esters of the present invention can be used to prepare pharmaceutical compositions for preventing and treating virus infection, in particular for diseases associated with HPV infection in human and animals i.e. condyloma acuminata induced by HPV, cervical erosion in HPV positive patients, chronic cervicitis in HPV positive patients, or to aid in the treatment of cervical cancer.

The hydroxybenzoic acid esters disclosed in the present invention are more effective and stable than the known hydroxybenzoic acid and its analogues. The hydroxybenzoic acid ester and its analogues can be made into various medicines and personal care products when prepared together with pharmaceutical adjuvants.

The present invention has been described above in detail in general terms and specific examples. A person skilled in the art can easily make some modifications or improvements based on the description in this application. Therefore, the relevant modifications and improvements based on the present invention are within the scope of the present invention.

## Claims

1. The use of hydroxybenzoic acid ester as set in Formula (I) below in the manufacture of a medicament for the prevention and treatment of human or animal HPV infection, condyloma acuminata induced by HPV, cervical erosion in HPV positive patients, chronic cervicitis in HPV positive patients, or to aid in the treatment of cervical cancer, wherein R is C1-11 alkyl group; R1, R2, R3, R4 and R5 are independently OH or H; and at least two of which are OH.

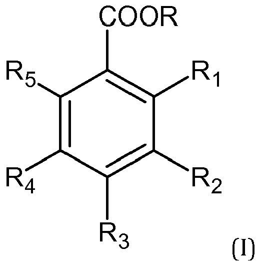

2. The use of a hydroxybenzoic acid ester in the manufacture of a medicament according to claim 1 wherein said R is a C₁₋₈ alkyl group.

3. The use of a hydroxybenzoic acid ester in the manufacture of a medicament according to claims 1 or 2, wherein said R is a C₁₋₃ alkyl group.

4. The use of a hydroxybenzoic acid ester in the manufacture of a medicament according to any of claims 1 to 3, wherein at least three of said R₁, R₂, R₃, R₄ and R₅ are OH.

5. The use of a hydroxybenzoic acid ester in the manufacture of a medicament according to any of claims 1 to 3, wherein said hydroxybenzoic acid ester is selected from the group consisting of 2, 4-dihydroxybenzoic acid ester, 3, 4-dihydroxybenzoic acid ester, 2, 3, 4-trihydroxybenzoic acid ester, 3, 4, 5-trihydroxybenzoic acid ester and 3, 4, 6-trihydroxybenzoic acid ester.

6. The use of a hydroxybenzoic acid ester in the manufacture of a medicament according to any of claims 1 to 5, wherein said hydroxybenzoic acid ester is selected from the group consisting of 2, 3, 4-trihydroxybenzoic acid ester, 3, 4, 5-trihydroxybenzoic acid ester and 3, 4, 6-trihydroxybenzoic acid ester.

7. The use of a hydroxybenzoic acid ester in the manufacture of a medicament according to any of claims 1 to 6, wherein said hydroxybenzoic acid ester is 3, 4, 5-trihydroxybenzoic acid ester.

8. The use of a hydroxybenzoic acid ester in the manufacture of a medicament according to any of claims 1 to 7, wherein said compound and/or a composition comprising such compound as an active ingredient are/is made into powder, solution, tablet, capsule, granule or liquid with various adjuvants for injection or oral formulation or made into ointment, gel, lotion, tablet, suppository, diaphragm or spray with various adjuvants for skin mucosa formulation, or other pharmaceutically acceptable formulation.

9. The use of a hydroxybenzoic acid ester in the manufacture of a medicament according to any of claims 1 to 7, wherein said compound and/or composition comprising such compound as an active ingredient are/is used as personal care products, wherein such personal care products are hand soaps, hand sanitizers, body cleansers, body cleansing gels, body washing soaps, personal care wipes, facial tissues, nasal sprays or combinations thereof.

## Patentansprüche

1. Verwendung von Hydroxybenzoesäureester gemäß nachstehender Formel (1) bei der Herstellung eines Medikaments zur Prävention und Behandlung von menschlicher oder tierischer HPV-Infektion, von durch HPV induzierter Condolymata acuminata, von Portioerosion bei HPV-positiven Patienten, von chronischer Zervizitis bei HPV-positiven Patienten oder zur Unterstützung bei der Behandlung von Gebärmutterhalskrebs, wobei R eine C1-11-Alkylgruppe ist, R1, R2, R3, R4 und R5 unabhängig OH oder H sind, und wobei mindestens zwei davon OH sind.

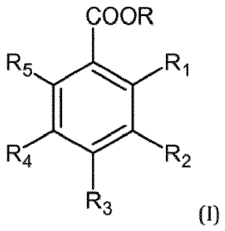

2. Verwendung von Hydroxybenzoesäureester bei der Herstellung eines Medikaments nach Anspruch 1,
wobei R eine C₁₋₈-Alkylgruppe ist.

3. Verwendung von Hydroxybenzoesäureester bei der Herstellung eines Medikaments nach Anspruch 1 oder 2,
wobei R eine C₁₋₃-Alkylgruppe ist.

4. Verwendung von Hydroxybenzoesäureester bei der Herstellung eines Medikaments nach einem der Ansprüche 1 bis 3,
wobei von R₁, R₂, R₃, R₄ und R₅ mindestens drei OH sind.

5. Verwendung von Hydroxybenzoesäureester bei der Herstellung eines Medikaments nach einem der Ansprüche 1 bis 3,
wobei der Hydroxybenzoesäureester aus der Gruppe bestehend aus 2,4-Dihydroxybenzoesäureester, 3,4-Dihydroxybenzoesäureester, 2,3,4-Trihydroxybenzoesäureester, 3,4,5-Trihydroxybenzoesäureester und 3,4,6-Trihydroxybenzoesäureester ausgewählt ist.

6. Verwendung von Hydroxybenzoesäureester bei der Herstellung eines Medikaments nach einem der Ansprüche 1 bis 5,
wobei der Hydroxybenzoesäureester aus der Gruppe bestehend aus 2,3,4-Trihydroxybenzoesäureester, 3,4,5-Trihydroxybenzoesäureester und 3,4,6-Trihydroxybenzoesäureester ausgewählt ist.

7. Verwendung von Hydroxybenzoesäureester bei der Herstellung eines Medikaments nach einem der Ansprüche 1 bis 6,
wobei der Hydroxybenzoesäureester 3,4,5-Trihydroxybenzoesäureester ist.

8. Verwendung von Hydroxybenzoesäureester bei der Herstellung eines Medikaments nach einem der Ansprüche 1 bis 7,
wobei die Verbindung und/oder eine solche Verbindung umfassende Zusammensetzung zu Pulver, einer Lösung, Tabletten, Kapseln, Granulat oder einer Flüssigkeit mit verschieden Hilfsmitteln für eine Injektion oder orale Rezeptur verarbeitet werden/wird oder zu Salbe, Gel, Lotion, Tabletten, Zäpfchen, Diaphragmen oder Spray mit verschiedenen Hilfsmitteln für eine Schleimhautrezeptur oder eine andere pharmazeutisch annehmbare Rezeptur verarbeitet werden/ wird.

9. Verwendung von Hydroxybenzoesäureester bei der Herstellung eines Medikaments nach einem der Ansprüche 1 bis 7,
wobei die Verbindung und/oder eine solche Verbindung umfassende Zusammensetzung als Körperpflegemittel verwendet werden/wird, wobei solche Körperpflegemittel Handseifen, Händedesinfektionsmittel, Körperreinigungsmittel, Körperreinigungsgels, Körperreinigungsseifen, Körperpflegetücher, Gesichtstücher, Nasensprays oder Kombinationen davon sind.

## Revendications

1. Utilisation d'un ester d'acide hydroxybenzoique tel qu'indiqué dans la Formule (I) ci-dessous dans la fabrication d'un médicament pour la prévention et le traitement d'une infection par un papillomavirus (HPV) humain ou animal, du condylome acuminé induit par le HPV, de l'érosion du col de l'utérus chez les patientes positives au HPV, de la cervicite chronique chez les patientes positives au HPV, ou pour aider dans le traitement du cancer du col de l'utérus, où R représente un groupe alkyle en C₁-C₁₁ ; R1, R2, R3, R4 et R5 représentent indépendamment OH ou H ; et dont au moins deux représentent OH.

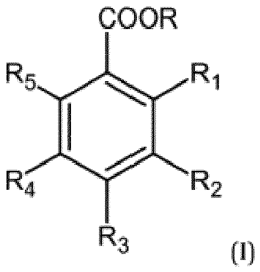

2. Utilisation d'un ester d'acide hydroxybenzoïque dans la fabrication d'un médicament selon la revendication 1,
dans laquelle ledit R représente un groupe alkyle en C₁-C₈.

3. Utilisation d'un ester d'acide hydroxybenzoïque dans la fabrication d'un médicament selon l'une des revendications 1 ou 2,
dans laquelle ledit R représente un groupe alkyle en C₁-C₃.

4. Utilisation d'un ester d'acide hydroxybenzoïque dans la fabrication d'un médicament selon l'une quelconque des revendications 1 à 3,
dans laquelle au moins trois desdits R1, R2, R3, R4 et R5 représentent OH.

5. Utilisation d'un ester d'acide hydroxybenzoïque dans la fabrication d'un médicament selon l'une quelconque des revendications 1 à 3,
dans laquelle ledit ester d'acide hydroxybenzoïque est choisi dans le groupe consistant en ester de l'acide 2,4-dihydroxybenzoïque, ester de l'acide 3,4-dihydroxybenzoïque, ester de l'acide 2,3,4-trihydroxybenzoïque, ester de l'acide 3,4,5-trihydroxybenzoïque et ester de l'acide 3,4,6-trihydroxybenzoïque.

6. Utilisation d'un ester d'acide hydroxybenzoïque dans la fabrication d'un médicament selon l'une quelconque des revendications 1 à 5,
dans laquelle ledit ester d'acide hydroxybenzoïque est choisi dans le groupe consistant en ester de l'acide 2,3,4-trihydroxybenzoïque, ester de l'acide 3,4,5-trihydroxybenzoïque et ester de l'acide 3,4,6-trihydroxybenzoïque.

7. Utilisation d'un ester d'acide hydroxybenzoïque dans la fabrication d'un médicament selon l'une quelconque des revendications 1 à 6,
dans laquelle ledit ester d'acide hydroxybenzoïque est un ester de l'acide 3,4,5-trihydroxybenzoïque.

8. Utilisation d'un ester d'acide hydroxybenzoïque dans la fabrication d'un médicament selon l'une quelconque des revendications 1 à 7,
dans laquelle ledit composé et/ou une composition comprenant un tel composé comme principe actif est transformé en une poudre, une solution, un comprimé, une capsule, un granulé ou un liquide avec divers adjuvants pour une formulation pour injection ou pour une formulation orale ou est transformé en une pommade, un gel, une lotion, un comprimé, un suppositoire, un diaphragme ou une pulvérisation avec divers adjuvants pour une formulation pour les muqueuses de la peau, ou une autre formulation pharmaceutiquement acceptable.

9. Utilisation d'un ester d'acide hydroxybenzoïque dans la fabrication d'un médicament selon l'une quelconque des revendications 1 à 7,
dans laquelle ledit composé et/ou ladite composition comprenant un tel composé comme principe actif est utilisé comme produits de soins personnels, ces produits de soins personnels étant les savons pour les mains, les produits d'hygiène pour les mains, les nettoyants pour le corps, les gels nettoyants pour le corps, les savons de lavage pour le corps, les lingettes de soins personnels, les mouchoirs en papier, les pulvérisations nasales ou leurs combinaisons.
